# EUROPÄISCHE PATENTSCHRIFT

(11) **EP 1 265 662 B1**
(45) Veröffentlichungstag und Bekanntmachung des Hinweises auf die Patenterteilung: **07.09.2005**
(21) Anmeldenummer: 00988555.9
(22) Anmeldetag: 31.10.2000
(51) Int. Cl.: A61M 5/148, B25B 33/00

(54) **VORRICHTUNG ZUR VERABREICHUNG VON FLÜSSIGKEITEN**
DEVICE FOR ADMINISTERING LIQUIDS
DISPOSITIF D'ADMINISTRATION DE LIQUIDES

(43) Veröffentlichungstag der Anmeldung: 18.12.2002
(60) Teilanmeldung: 05000671.7
(73) Patentinhaber: Onkoworks GmbH f. Herstellung und Vertrieb Onkologischer Spezialpräparate, 42781 Haan (DE)
(72) Erfinder: FISCHER, Wilfried, 42781 Haan (DE); BRÜCKER, Matthias, CH-9200 Gossau (CH)
(74) Vertreter: Hauck, Graalfs, Wehnert, Döring, Siemons
(86) Internationale Anmeldenummer: PCT/DE2000/003837
(87) Internationale Veröffentlichungsnummer: WO 2002/036185

(56) Entgegenhaltungen:
- WO-A-97/34651
- DE-C- 318 761
- FR-A- 2 569 675
- FR-A- 2 677 887
- GB-A- 2 054 381
- US-A- 838 104

## Beschreibung

Die vorliegende Erfindung betrifft eine Vorrichtung zur Verabreichung von Flüssigkeiten, insbesondere Infusionslösungen.

Derartige Vorrichtungen sind bekannt. Ein Anwendungsfall einer derartigen Vorrichtung betrifft beispielsweise die Verabreichung einer Infusionslösung über einen langen Zeitraum, wobei die Infusionslösung in relativ geringen Mengen über den entsprechenden Zeitraum verabreicht werden soll. Eine solche Vorrichtung wird auch als "Infusionspumpe" bezeichnet. Sie sorgt dafür, daß die beispielsweise in einem Infusionsbeutel enthaltene Infusionsflüssigkeit aus dem Infusionsbeutel über ein Schlauchsystem dem Körper des Patienten zugeführt wird.

Aus der DE 295 08 249 U1 ist eine Vorrichtung zum Verabreichen von medizinischen Flüssigkeiten mit einem mit der zu verabreichenden Flüssigkeit gefüllten faltenbalgartigen Behälter bekannt. Dieser faltenbalgartige Behälter ist in ein im wesentlichen zylindrisches Gehäuse eingesetzt, das mit einem kappenartigen, die Gehäusewand übergreifenden Deckel verschlossen ist. Im Gehäusedeckel befindet sich eine den faltenbalgartigen Behälter mit einer Druckkraft beaufschlagende Druckfeder. Deckel und Gehäusewand wirken über ein Gewinde zusammen, so daß mit zunehmendem Aufschrauben des Deckels auf das Gehäuse der faltenbalgartige Behälter zunehmend unter Druck gesetzt wird und hierdurch die darin enthaltene zu verabreichende Flüssigkeit abgegeben wird.

Nachteilig ist bei dieser bekannten Vorrichtung, daß mit zunehmender Entleerung des faltenbalgartigen Behälters der Deckel weitergeschraubt werden muß. Der Verabreichungsvorgang läuft daher nicht selbsttätig ab.

Aus der GB-A-2054 381 ist eine Vorrichtung gemäß dem Oberbegriff des Anspruch 1 bekannt.

Der vorliegenden Erfindung liegt die Aufgabe zugrunde, eine Vorrichtung zur Verabreichung von Flüssigkeiten zu schaffen, die bei einer selbsttätigen Funktionsweise bequem und einfach handhabbar bzw. anwendbar ist sowie eine gleichmäßige Dosierung der zu verabreichenden Flüssigkeit ermöglicht.

Diese Aufgabe wird erfindungsgemäß durch eine Vorrichtung gemäß Anspruch 1 gelöst.

Die erfindungsgemäße Lösung sieht ein Gehäuse vor, das eine untere und eine obere Gehäusehälfte aufweist, die schwenkbar bzw. drehbar miteinander verbunden sind. Ein Verschluß hält beide Gehäusehälften im geschlossenen Zustand zusammen. Zum Einlegen eines die Flüssigkeit (Infusionslösung) aufnehmenden Beutels ist der Verschluß öffenbar, wonach die obere Gehäusehälfte aufgeklappt werden kann. Nach eingelegtem Beutel wird die obere Gehäusehälfte in den geschlossenen Zustand zurückgeschwenkt, wonach der Verschluß beide Gehäusehälften miteinander verriegelt. Der im Inneren des Gehäuses angeordnete Druckstempel übt im geschlossenen Zustand des Gehäuses eine Kraft auf den gefüllten Beutel aus, die die Abgabe der Flüssigkeit vom Beutel, beispielsweise über ein mit dem Beutel in Verbindung stehendes Schlauchsystem, sicherstellt. Damit der Druckstempel eine solche Kraft aufbringen kann, wird er selbst über einen Mechanismus zur Druckaufbringung beaufschlagt, und zwar derart, daß ein Druck im Beutel erzeugt wird, der die Abgabe eines konstanten Flüssigkeitsvolumens pro Zeiteinheit über den Zeitraum des Verabreichungsprozesses bewirkt. Mit anderen Worten, der Durchsatz (Flow Rate) der Flüssigkeit durch einen an den Beutel angeschlossenen Schlauch wird über den Verabreichungsprozeß konstant gehalten.

Der erfindungsgemäße Gedanke, anstelle eines einfachen Druckfedersystems zur Beaufschlagung eines die zu verabreichende Flüssigkeit enthaltenden Beutels einen solchen Mechanismus einzusetzen, stellt einen der zentralen Punkte der Erfindung dar. Dieser Mechanismus schaltet folgendes Phänomen aus. Bei vollem Beutel, d.h. wenn die Kraft, um einen bestimmten Druck zu erzeugen, nicht so groß sein muß, ist eine Feder gerade am stärksten gespannt. Bei leerem Beutel hingegen, wenn die Kraft, um einen bestimmten Druck zu erzeugen, sehr hoch sein muß, ist die Feder fast nicht gespannt. Erfindungsgemäß wird sichergestellt, daß trotz dieser Federeigenschaten die Abgabe eines konstanten Flüssigkeitsvolumens pro Zeiteinheit erreicht wird.

Der Mechanismus weist einen den Druckstempel mit einer Kraft beaufschlagenden, durch eine Zugfeder in seiner Winkelstellung relativ zum Druckstempel veränderbaren Hebel auf. Die Kraftaufbringung auf den Druckstempel erfolgt somit durch Veränderung der Winkelstellung eines Hebels, der bei entleertem Beutel nahezu rechtwinklig zur Druckstempelebene angeordnet ist, während er bei vollständig gefülltem Beutel eine Lage nahezu parallel zur Druckstempelebene einnimmt. Mit zunehmendem Entleeren des Beutels nimmt der Winkel, den der Hebel mit der Druckstempelebene bildet, zu. Hierdurch wird ein gleichmäßiger Druckaufbau im Beutel während der Verabreichungszeit sichergestellt.

Speziell besitzt der Mechanismus vorzugsweise einen an einem Ende drehbar und verschiebbar an der oberen Gehäusehälfte gelagerten und am anderen Ende den Druckstempel drehbar lagernden Kipphebel und eine an dem einen Ende des Kipphebels über ein flexibles Seil angreifende Zugfeder, die den Kipphebel in die der untersten Druckstempellage entsprechende Kippstellung vorspannt. Wenn kein Beutel in das Gehäuse eingelegt ist, nimmt der Druckstempel seine unterste Lage ein, die der am stärksten gekippten Stellung des Kipphebels entspricht. Wird ein Beutel eingelegt und das Gehäuse verschlossen, wird der Kipphebel in eine nahezu zur Druckstempelebene parallele Lage verschwenkt, in der die Zugfeder am weitesten ausgelängt ist. Mit fortschreitendem Entleeren des Beutels wird die Zugfeder zunehmend entspannt und die Kippstellung des Hebels verändert. Gleichzeitig wird hierdurch der Hebelarm (Abstand zwischen Federangriffspunkt und Stempeldrehpunkt am Kipphebel) verändert, mit dem die Zugfeder die Kraft auf den Druckstempel überträgt. Bei vollem Beutel (Lage des Kipphebels etwa parallel zum Druckstempel) ist der Hebelarm sehr kurz, während bei leerem Beutel (Lage des Kipphebels stark geneigt) der Hebelarm sehr lang ist. Durch Veränderung der Hebelverhältnisse wird somit die unterschiedliche Federkraft kompensiert.

Vorzugsweise ist das flexible Seil über die Oberseite des Kipphebels geführt und die Oberseite in einer vorbestimmten Kontur, insbesondere etwa dachförmig, ausgebildet, wobei das Seil, je nach Winkelstellung des Kipphebels, unterschiedlich stark umgelenkt wird. Mit dieser Maßnahme wird die erfindungsgemäße Zielsetzung noch besser erreicht, da der Kipphebel hierbei als Exzenter wirkt, dessen Kontur die Hebelverhältnisse festlegt. Das flexible Seil läuft hierbei über die Kontur auf der Oberseite des Kipphebels ab, so daß der Hebelarm durch die jeweilige Kontaktstelle des Seils mit der Kontur (vorzugsweise Dachform) festgelegt wird.

Die Zugfeder ist an der Innenseite der oberen Gehäusehälfte verankert. Es versteht sich von selbst, daß der Verankerungspunkt der Zugfeder in bezug auf das eine Kipphebelende, an dem die Zugfeder befestigt ist, jenseits des anderen Kipphebelendes (drehbare Verbindung mit dem Zugstempel) liegt.

Je nach Anwendungsfall sind Federcharakteristik, Hebelverhältnis, Hebelform etc. empirisch zu ermitteln, um die geeigneten Kräfteverhältnisse zu erhalten.

Um etwa symmetrische Verhältnisse zu schaffen, weist die Vorrichtung vorzugsweise mindestens zwei entgegengesetzt zueinander wirkende Kipphebel mit zugehörigen entgegengesetzt wirkenden Zugfedern auf, wobei die Kipphebel an ihren jeweiligen anderen Enden eine gemeinsame Drehachse am Druckstempel besitzen. In Weiterbildung dieser Ausgestaltung weist die Vorrichtung zwei innenliegende, in einer Richtung wirkende Kipphebel und zwei außenliegende, in der entgegengesetzten Richtung wirkende Kipphebel mit entsprechenden Zugfedern auf.

Die Kipphebel sind zweckmäßigerweise mittels Rollen auf Stegen verschiebbar, wobei die Rollen in ihrer Endstellung in komplementären konkaven Ausnehmungen der oberen Gehäusehälfte drehbar gelagert sind. Dabei weist jeder Kipphebel vorzugsweise an jeder Seite eine Rolle auf. Die Rollen besitzen in ihrem Umfang vorzugsweise eine Nut, in die der entsprechende Führungssteg für die Lagerung der Rolle eingreift. Auf diese Weise kann die Rolle auf dem Steg abrollen, und der Kipphebel ist gegen Bewegungen in Axialrichtung der Rollen gesichert.

Die zugehörige Zugfeder greift über das flexible Seil am oberen Ende des Kipphebels an. Das Seil ist im oberen Endbereich des Kipphebels fixiert, beispielsweise über eine Verdickung am Seilende dort eingelassen. Die Zugfeder übt somit über das Seil ein Drehmoment auf den Kipphebel aus, um diesen in die nahezu vertikale Kippstellung zu bringen. Diese Bewegung bewirkt ein Absenken des Druckstempels und damit ein Zusammenpressen des Beutels (Infusionsbeutels).

Das Gehäuse der erfindungsgemäßen Vorrichtung ist vorzugsweise flach ausgebildet, wobei eine Ausführungsform besonders bevorzugt wird, bei der das Gehäuse und der Druckstempel etwa rechteckig ausgebildet sind.

Die erfindungsgemäß ausgebildete Vorrichtung läßt sich bei entsprechender Ausbildung, wie vorstehend vorgeschlagen, bequem am Körper tragen, insbesondere mit Hilfe eines Gurtes unter einem Pullover etc. Das Gehäuse weist daher vorzugsweise auf seiner Rückseite mindestens eine Gurtlasche auf, durch die ein Gurt bzw. Gürtel gezogen werden kann. Durch die flache und insbesondere auch rechteckige Form ergibt sich ein hoher Tragekomfort.

Der Verschluß ist zweckmäßigerweise so ausgebildet, daß er mindestens eine an einer Gehäusehälfte angeordnete, die andere Gehäusehälfte beim Schließen außen übergreifende und mit der anderen Gehäusehälfte in Rasteingriff tretende Verschlußlasche aufweist. Vorzugsweise ist an jeder Seite eine Verschlußlasche vorgesehen.

Eine bevorzugte Ausführungsform zeichnet sich dadurch aus, daß die Vorrichtung ohne Zuhilfenahme von Spezialwerkzeug nicht öffenbar ist. Hierdurch soll verhindert werden, daß unerwünschte Manipulationen von Laien, beispielsweise vom Patienten selbst, durchgeführt werden können. Der Verschluß ist daher so ausgebildet, daß ein Öffnen des Gehäuses mit den Fingern nicht möglich ist.

Vielmehr ist ein spezielles Gerät zum Schließen und Öffnen der Vorrichtung der vorstehend beschriebenen Art vor gesehen. Dieses Gerät besitzt einen unteren stationären Lagerteil zur Fixierung der Vorrichtung und einen manuell handhabbaren, bügelartigen oberen Teil, der mit dem unteren Lagerteil schwenkbar verbunden ist und einen Abschnitt zum Pressen gegen die obere Gehäusehälfte der Vorrichtung besitzt. Das Gerät zum Schließen und Öffnen (Ladegerät) funktioniert so, daß die leere Vorrichtung geöffnet in das Gerät eingesetzt wird. Der gefüllte Beutel wird in die untere Gehäusehälfte der Vorrichtung eingelegt. Mit dem bügelartigen oberen Teil kann die obere Gehäusehälfte der Vorrichtung mit einem vernünftigen Kraftaufwand gegen die untere Gehäusehälfte gepreßt werden, bis der Verschluß einrastet. Die Vorrichtung ist dann betriebsbereit.

Zum Öffnen der Vorrichtung weist das Ladegerät vorzugsweise am oberen Teil eine Einrichtung zum Lösen der Verrastung der mindestens einen Verschlußlasche mit dem anderen Gehäuseteil auf. Die Einrichtung zum Lösen besitzt vorzugsweise ein die Verschlußlasche nach außen bewegendes keilförmiges Teil, das manuell nach unten bewegt werden kann, um die Verschlußlasche (die Verschlußlaschen) zu lösen. Die obere Gehäusehälfte schnappt dann nach oben, so daß die Vorrichtung dann offen ist. Sie kann aus dem Ladegerät entfernt und wieder mit einem neuen gefüllten Beutel beschickt werden.

Die Erfindung wird nachfolgend anhand eines Ausführungsbeispieles in Verbindung mit der Zeichnung im einzelnen erläutert. Es zeigen:
- Figur 1: eine räumliche Ansicht einer Vorrichtung zur Verabreichung von Flüssigkeiten im geöffneten Zustand, wobei die Federn, Zugseile und Lagereinrichtungen für die Kipphebel im von der oberen Gehäusehälfte gelösten Zustand dargestellt sind;
- Figuren 2a-f: schematische Schnittansichten der Vorrichtung der Figur 1, die die Funktionsweise des Mechanismus zur Druckaufbringung in einer stufenweise Abfolge zeigen; und
- Figur 3: eine räumliche Darstellung eines Ladegerätes zum Schließen und Öffnen der in den Figuren 1 und 2 gezeigten Vorrichtung.

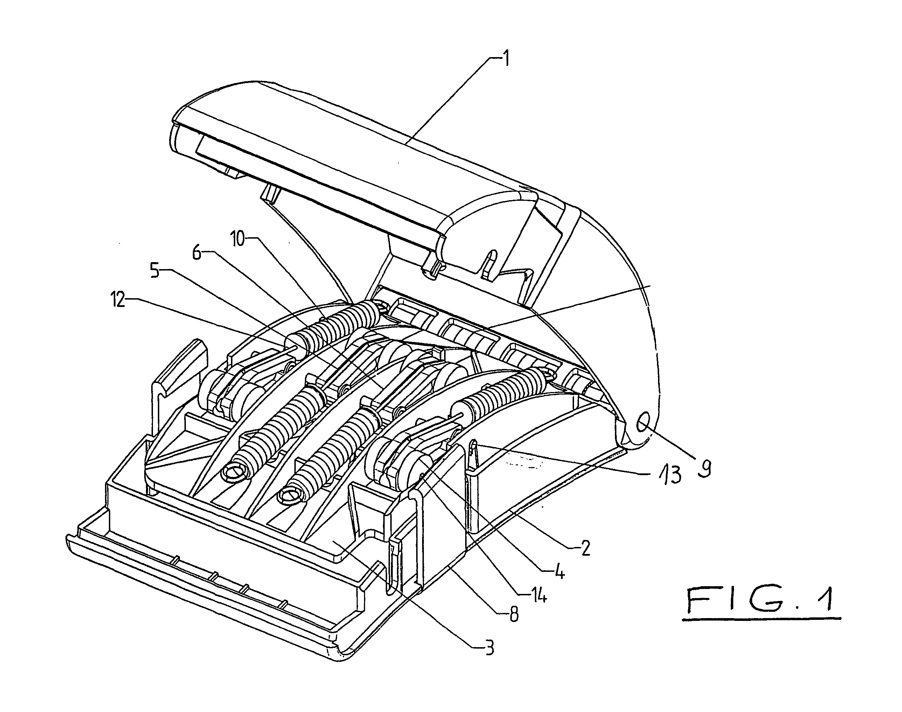

Wie man Figur 1 entnehmen kann, umfaßt die dargestellte Vorrichtung zur Verabreichung von Flüssigkeiten, bei der es sich im vorliegenden Fall um eine Infusionspumpe handelt, eine obere Gehäusehälfte 1 und eine untere Gehäusehälfte 2, die über ein bei 9 gezeigtes Scharnier schwenkbar miteinander verbunden sind. Beide Hälften bilden im geschlossenen Zustand ein Gehäuse, das eine flache, etwa rechteckige Form besitzt und einer Druckschnalle nahekommt. Auf der Rückseite der unteren Gehäusehälfte 2 sind zwei Gurtlaschen angeordnet, durch die ein Gurt gezogen werden kann, mit dem die Infusionspumpe am Körper getragen werden kann.

Wenn die obere Gehäusehälfte 1 zum Schließen verschwenkt wird, werden zwei seitlich an der unteren Gehäusehälfte 2 angeordnete Verschlußlaschen 8 etwas nach außen gedrückt und gleiten in einem entsprechenden Bereich der oberen Gehäusehälfte 1 nach oben, bis sie schließlich in einen entsprechenden Freiraum nach innen schnappen und somit beide Gehäusehälften fest miteinander verriegeln. Die Gehäusehälften lassen sich dann nicht mehr ohne weiteres öffnen, vielmehr wird hierzu ein später beschriebenes Spezialgerät benötigt.

Innerhalb des Gehäuses befindet sich ein Druckstempel 3, der etwa Rechteckform besitzt und geringfügig nach unten konkav gekrümmt ausgebildet ist. Der Druckstempel 3 ist über eine Drehachse 13, die etwa mittig am Druckstempel angeordnet ist, drehbar an vier Kipphebeln 5, 6 gelagert, welche wiederum an der Innenseite der oberen Gehäusehälfte 1 verschiebbar und drehbar gelagert sind. Wie man Figur 1 entnehmen kann, erstrecken sich die beiden inneren Kipphebel 5, 6 von der Drehachse 13 in Figur 1 nach rechts, während sich die beiden äußeren Kipphebel von der Drehachse 13 in der Figur nach links erstrecken. Es liegen somit etwa symmetrische Verhältnisse vor.

Die verschiebbare und drehbare Lagerung der vier Kipphebel an der oberen Gehäusehälfte ist mit Rollen 4 bewerkstelligt, wobei jeweils eine Rolle an einer Seite eines Kipphebels angeordnet ist. Diese Rollen 4 sind in komplementären Ausnehmungen in nach innen vorstehenden Abschnitten der Innenseite der oberen Gehäusehälfte 1 gelagert. Die Rollen 4 weisen um ihren Umfang herum mittlere Nuten (nicht gezeigt) auf, die mit entsprechenden Stegen (nicht gezeigt) an der Innenseite der oberen Gehäusehälfte 1 in Kontakt stehen. Die Rollen 4 können entlang dieser Stege abrollen..

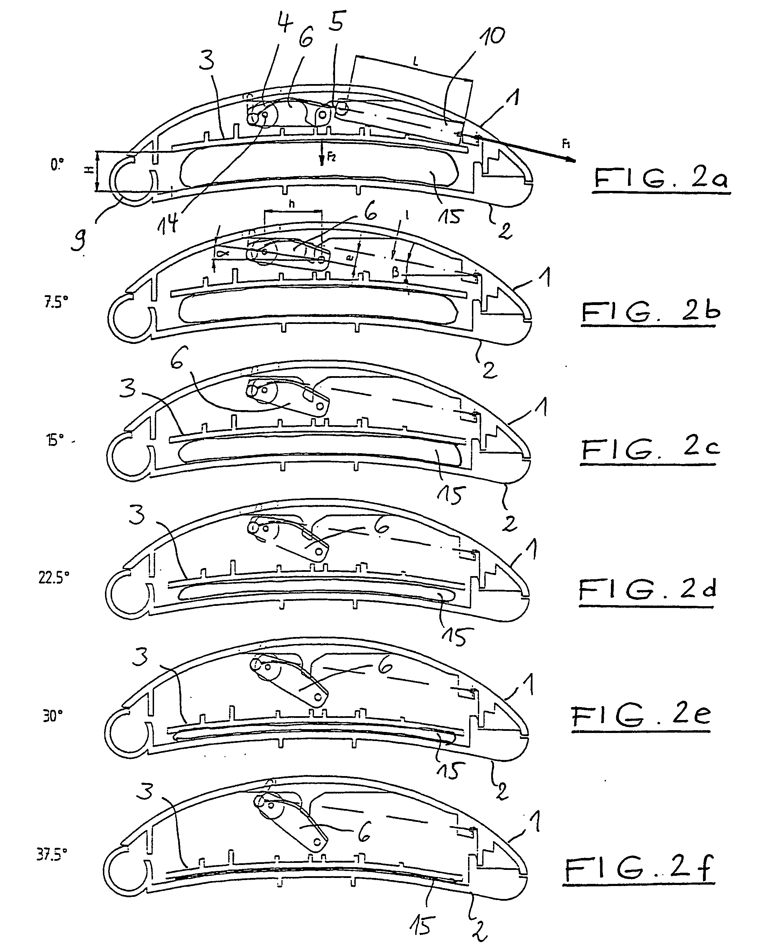

Die Kipphebel 6 stehen über flexible Zugseile 5 mit Zugfedern 10 in Verbindung, die die Kipphebel in ihrem an der Innenseite der oberen Gehäusehälfte 1 angeordneten Endbereich (Rollen) mit einer Zugkraft beaufschlagen. Die exakte Funktionsweise der Zugfedern und Kipphebel wird anhand Figur 2 erläutert.

Die Figuren 2a-f zeigen schematische Schnittansichten durch die Vorrichtung der Figur 1 zur Verdeutlichung der Funktionsweise des Mechanismus zur Druckaufbringung in stufenweiser Abfolge. Dabei ist in den Figuren jeweils nur ein Kipphebel 6 mit einer Zugfeder 10 dargestellt.

Figur 2a zeigt das aus den beiden Gehäusehälften 1 und 2 bestehende Gehäuse im geschlossenen Zustand bei eingelegtem gefüllten Infusionslösungsbeutel 15. Man erkennt, daß der Druckstempel 1 mit der nach unten gerichteten Kraft F₂ gegen den Beutel 15 gepreßt wird, und zwar mit Hilfe des Kipphebels 6, dessen in der Figur rechtes Ende drehbar am Druckstempel 3 gelagert ist. Das in der Figur linke Ende des Kipphebels 6 ist über bei 14 drehbar gelagerte Rollen 4 an der Innenseite der oberen Gehäusehälfte 1 verschiebbar und drehbar gelagert, wie vorstehend in Verbindung mit Figur 1 beschrieben wurde. An diesem Ende des Kipphebels 6 greift eine Zugfeder 10 über ein flexibles Zugseil 5 an. Die Zugfeder 10 hat eine Länge L und wirkt mit einer Zugkraft F₁, die in der Figur nach rechts gerichtet ist, auf den Kipphebel ein. Die Höhe des Beutels 15 ist mit H bezeichnet.

Man erkennt, daß der Kipphebel 6 mit der Horizontalen einen Winkel von 0° bildet, d.h. der Kipphebel 6 nimmt seine in die Horizontale verschwenkte Stellung ein, in der die Zugfeder 10 am weitesten ausgelängt ist. Man erkennt ferner, daß das flexible Zugseil 5 über die Oberseite des Kipphebels 6 geführt ist. Diese Oberseite ist etwa dachförmig ausgebildet, so daß der Kipphebel 5 als Exzenter wirkt, und lenkt das am in der Figur linken Ende des Kipphebels 6 fixierte Zugseil 5 um. Der Abstand zwischen der Kontaktstelle des Zugseils 5 mit der Oberseite des Kipphebels am Druckstempel entspricht dem Hebelarm, über den die Federkraft auf den Druckstempel einwirkt.

Figur 2b zeigt den Beutel 15 in einer bereits etwas entleerten Stellung. Hier hat sich der Druckstempel 3 bereits nach unten bewegt, und der Kipphebel 6 nimmt eine Stellung von 7,5° zur Horizontalen ein, wie mit dem Winkel α gekennzeichnet. Ferner sind in Figur 2b der horizontale Abstand zwischen den Drehpunkten mit h, der Abstand zwischen Drehpunkt des Kipphebels am Druckstempel 3 und der Kontaktstelle des Zugseils 5 (Hebelarm) mit e, die Zugrichtung der Feder mit β und die Länge der Feder mit 1 bezeichnet.

Die Figuren 2c-2f zeigen weitere Stufen in der Funktionsweise des Mechanismus, wobei Figur 2f die tiefste Stellung des Druckstempels 3 zeigt, in der der Kipphebel 6 einen Winkel von 37,5° mit der Horizontalen bildet. In dieser Stellung ist die Zugfeder nahezu ungespannt.

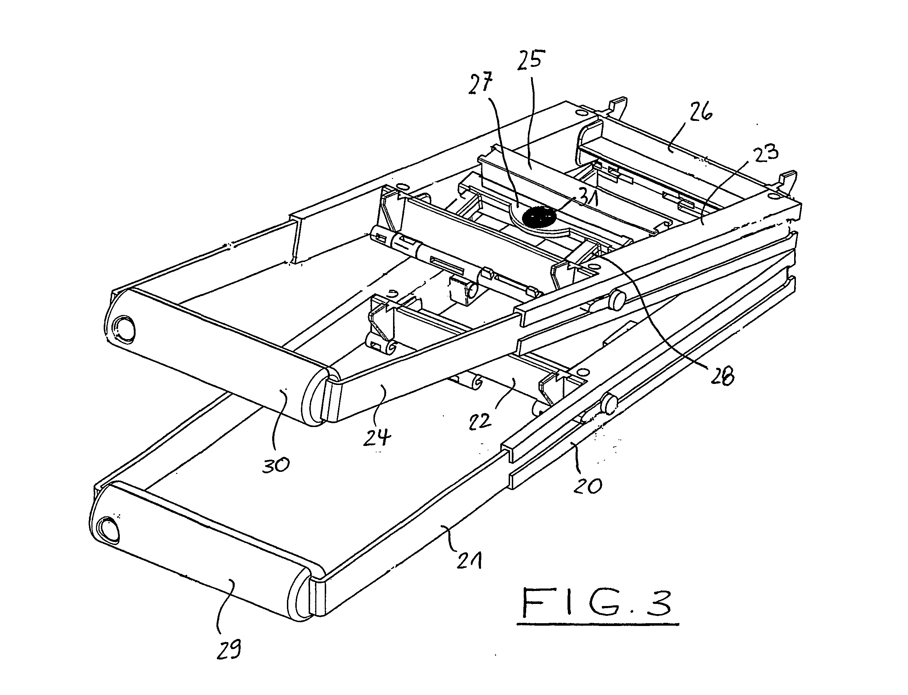

Figur 3 zeigt ein Gerät zum Öffnen und Schließen der in den Figuren 1 und 2 gezeigten Vorrichtung. Das Gerät besitzt ein rahmenförmiges unteres Teil 20, das einen ausziehbaren Teil 21 mit einem vorderen Griff 29 besitzt. Das rahmemförmige untere Teil 20 bildet ein Lager für die zu öffnende oder zu schließende Vorrichtung, die dort zwischen den beiden Längsschenkeln sowie dem vorderen Querschenkel 22 und dem hinteren Querschenkel des rahmenförmigen Unterteiles 20 fixiert werden kann.

Das untere Lagerteil 20 ist schwenkbar mit einem oberen Teil 23 verbunden, das bügelartig ausgebildet ist und ebenfalls einen ausziehbaren Teil 24 aufweist, an dessen Vorderseite ein Handgriff 30 angeordnet ist. Mit Hilfe der Griffe 29 und 30 können die beiden Teile 20 und 23 auf eine geeignete Länge gebracht werden. Das bügelartige Oberteil 23 wird nach dem Einlegen der zu schließenden Vorrichtung nach unten bewegt, wobei ein zwischen den Längsschenkeln des Teiles 23 angeordneter Querabschnitt 25, der drehbeweglich angeordnet ist, gegen die Oberseite der oberen Gehäusehälfte der Vorrichtung gepreßt wird. Auf diese Weise wird die Vorrichtung geschlossen.

Zwischen den Längsschenkeln des Oberteiles 23 ist ferner eine Öffnungseinrichtung 27, d.h. eine Einrichtung zum Lösen der Verrastung der mindestens einen Verschlußlasche mit dem anderen Gehäuseteil der Vorrichtung vorgesehen. Diese Öffnungseinrichtung 27 hat einen Querbalken mit einem bei 31 angedeuteten Preßabschnitt, an dem mittels Fingerdruck der Querbalken nach unten bewegt wird. In den beiden seitlichen Endbereichen des Querbalkens sind keilförmige Abschnitte 28 angeordnet, die bei der Abwärtsbewegung die an der oberen Gehäusehälfte der zu öffnenden Vorrichtung angreifenden Verschlußlaschen seitlich nach außen pressen, so daß die Verrastung zwischen beiden Gehäusehälften gelöst wird. Hierdurch schnappt die obere Gehäusehälfte der Vorrichtung etwas nach oben und kann somit manuell geöffnet werden. Die Vorrichtung kann dann aus dem Ladegerät entnommen werden, um den verbrauchten Beutel zu entfernen und einen neuen Beutel einzulegen.

Mit anderen Worten, die Verriegelung (Verschlußlasche) ist manuell nicht lösbar, da durch den sehr hohen Federdruck im Inneren die Verschlußlasche so stark in die Kerbe am Gehäuseoberteil gepreßt wird, daß die Verschlußlasche bei bestehendem Innendruck nur sehr schwer aus der Rastpostion gelöst werden kann. Das Ladegerät dient nun dazu, mittels des Querabschnitts 25 und des ausziehbaren Hebels 24 diesem Innendruck entgegenzuwirken, damit die Verschlußlaschen aus der Rastposition freigegeben werden und so mit der Öffnungseinrichtung 27 die Laschen ohne viel Kraftaufwand auf die Seite geschoben werden können.

## Patentansprüche

1. Vorrichtung zur Verabreichung von Flüssigkeiten, insbesondere Infusionslösungen, mit
einer unteren Gehäusehälfte (2),
einer mit der unteren Gehäusehälfte (2) verbundenen oberen Gehäusehälfte (1),
einem die Gehäusehälften (1, 2) im geschlossenen Zustand zur Ausbildung eines Gehäuses zusammenhaltenden Verschluß und
einem Mechanismus zur Beaufschlagung eines zwischen die untere Gehäusehälfte (2) und den Mechanismus gelegten, mit der zu verabreichenden Flüssigkeit gefüllten Beutels (15), der bei geschlossenem Gehäuse den eingelegten Beutel (15) mit einer solchen Kraft beaufschlagt, daß ein Druck im Beutel (15) erzeugt wird, der die Abgabe eines konstanten Flüssigkeitsvolumens pro Zeiteinheit über den Zeitraum des Verabreichungsprozesses bewirkt, wobei der Mechanismus einen den Beutel (15) mit einer Kraft beaufschlagenden, durch eine Zugfeder (10) in seiner Winkelstellung relativ zum Beutel (15) veränderbaren Kipphebel (6) aufweist,
**dadurch gekennzeichnet, daß**
die obere Gehäusehälfte (1) verschwenkbar mit der unteren Gehäusehälfte (2) verbunden ist,
der Mechanismus einen im Gehäuse angeordneten Druckstempel (3) beaufschlagt, zwischen dem und der unteren Gehäusehälfte (2) der Beutel (15) angeordnet ist, wobei die Zugfeder (10) der untersten Druckstempellage entsprechende den kipphebel in seine Kippstellung vorspannt, und
der Kipphebel (6) an einem Ende drehbar und verschiebbar an der oberen Gehäusehälfte (1) gelagert ist und am anderen Ende den Druckstempel (3) drehbar lagert.

2. Vorrichtung nach Anspruch 1, **dadurch gekennzeichnet, daß** die Zugfeder (10) an dem einen Ende des Kipphebels (6) über ein flexibles Seil (5) angreift.

3. Vorrichtung nach Anspruch 2, **dadurch gekennzeichnet, daß** das flexible Seil (5) über die Oberseite des Kipphebels (6) geführt ist und die Oberseite in einer vorbestimmten Kontur, insbesondere etwa dachförmig, ausgebildet ist, wobei das Seil, je nach Winkelstellung des Kipphebels (6), unterschiedlich stark umgelenkt wird.

4. Vorrichtung nach einem der vorangehenden Ansprüche, **dadurch gekennzeichnet, daß** sie mindestens zwei entgegengesetzt zueinander wirkende Kipphebel (6) mit zugehörigen entgegengesetzt wirkenden Zugfedern (10) aufweist, wobei die Kipphebel (6) an ihren jeweiligen anderen Enden eine gemeinsame Drehachse (13) am Druckstempel (3) besitzen.

5. Vorrichtung nach Anspruch 4, **dadurch gekennzeichnet, daß** sie zwei in bezug auf die gemeinsame Drehachse (13) innenliegende, von der gemeinsamen Drehachse (13) aus in einer Richtung wirkende Kipphebel (6) und zwei außenliegende, in der entgegengesetzten Richtung wirkende Kipphebel (6) mit entsprechenden Zugfedern (10) aufweist.

6. Vorrichtung nach einem der vorangehenden Ansprüche, **dadurch gekennzeichnet, daß** die Kipphebel (6) mittels Rollen (4) auf Stegen verschiebbar sind, wobei die Rollen (4) in ihrer Endstellung in komplementären konkaven Ausnehmungen der oberen Gehäusehälfte (1) drehbar gelagert sind.

7. Vorrichtung nach einem der vorangehenden Ansprüche, **dadurch gekennzeichnet, daß** sie ein flaches Gehäuse aufweist.

8. Vorrichtung nach einem der vorangehenden Ansprüche, **dadurch gekennzeichnet, daß** das Gehäuse und der Druckstempel (3) etwa rechteckig ausgebildet sind.

9. Vorrichtung nach einem der vorangehenden Ansprüche, **dadurch gekennzeichnet, daß** der Druckstempel (3) in Richtung seiner Längsachse zur Anpassung an den die zu verabreichende Flüssigkeit enthaltenen Beutel (15) nach unten hin konkav ausgebildet ist.

10. Vorrichtung nach einem der vorangehenden Ansprüche, **dadurch gekennzeichnet, daß** das Gehäuse auf seiner Rückseite mindestens eine Gurtlasche aufweist.

11. Vorrichtung nach einem der vorangehenden Ansprüche, **dadurch gekennzeichnet, daß** der Verschluß mindestens eine an einer Gehäusehälfte (1, 2) angeordnete, die andere Gehäusehälfte beim Schließen außen übergreifende und mit der anderen Gehäusehälfte in Rasteingriff tretende Verschlußlasche (8) aufweist.

## Claims

1. A device for administering liquids, especially infusion solutions, comprising
a lower casing half (2),
an upper casing half (1) connected with the lower casing half (2),
a closure holding together the casing halfs (1, 2) in a closed condition for the formation of a casing and
an application mechanism for a bag (15) laid between the lower casing half (2) and the mechanism and filled with the liquid to be administered, said application mechanism applying such a force to the laid in bag (15) when the casing is closed that a pressure is generated in the bag (15) which causes the discharge of a constant liquid volume per unit of time over the period of time of the
administering process, wherein the mechanism comprises a rocking lever (6) applying a force to the bag (15) and variable in its angular position relative to the bag (15) by a tension spring (10),
**characterized in that**
the upper casing half (1) is pivotally connected with the lower casing half (2),
the mechanism acts on a pressure piston (3) located in the casing, the bag (15) being located between the pressure piston and the lower casing half (2), wherein the tension spring (10) biasses the rocking lever (6) into its tilting position corresponding to the lowermost position of the pressure pistion, and
the rocking lever (6) is pivotally and displaceably supported at the upper casing half (1) with one end and pivotally supports the pressure piston (3) with the other end.

2. The device according to claim 1, **characterized in that** the tension spring (10) acts upon the one end of the rocking lever (6) by means of a flexible cable (5).

3. The device according to claim 2, **characterized in that** the flexible cable (5) is guided over the upper side of the rocking lever (6) and that the upper side is formed in a predetermined contour, especially approximately in a roof-like manner, wherein the cable, dependent on the angle of position of the rocking lever (6), is turned round differently.

4. The device according to one of the preceding claims, **characterized in that** it comprises at least two rocking levers (6) acting oppositely with respect to one another and having associated oppositely acting tension springs (10), wherein the rocking levers (6) have a common pivot axis (13) at their respective other ends at the pressure piston (3).

5. The device according to claim 4, **characterized in that** it includes, with regard to the common pivot axis (13), two inwardly positioned rocking levers (6) acting in one direction from the common pivot axis (13) and two outwardly positioned rocking levers (6) acting in the opposite direction with corresponding tension springs (10).

6. The device according to one of the preceding claims, **characterized in that** the rocking levers (6) are adapted to be displaced on webs by means of rollers (4) wherein the rollers (4) in their end position are rotatably supported in complementary concave recesses of the upper casing half (1).

7. The device according to one of the preceding claims, **characterized in that** it includes a flat casing.

8. The device according to one of the preceding claims, cahracterized in that the casing and the pressure piston (3) are formed approximately rectangularly.

9. The device according to one of the preceding claims, **characterized in that** the pressure piston (3) is concavely formed downwardly in the direction of its longitudinal axis for the adaption to the bag (15) containing the the liquid which is to be administered.

10. The device according to one of the preceding claims, **characterized in that** the casing has at least one belt buckle on its backside.

11. The device according to one of the preceding claims, **characterized in that** the closure has at least one closing tongue (8) located at one casing half (1, 2), outwardly engaging the other casing half during closing and lockingly engaging the other casing half.

## Revendications

1. Dispositif d'administration de liquides, en particulier de solutions de perfusion, comprenant
une moitié inférieure (2) de logement,
une moitié supérieure (1) de logement reliée à la moitié inférieure (2) de logement,
une fermeture maintenant les moitiés (1, 2) de logement à l'état fermé pour former un logement, et
un mécanisme pour agir sur une poche (15) remplie du liquide à administrer, placée entre la moitie inférieure (2) de logement et le mécanisme qui, lors que le logement est fermé, agit sur la poche (15) mise en place avec une force telle qu'une pression est engendrée dans la poche (15), pression qui a pour effet la délivrance d'un volume constant de liquide par unité de temps au cours de la période de temps du processus d'administration, le mécanisme présentant un levier oscillant (6) agissant sur la poche (15) avec une force, modifiable dans sa position angulaire par rapport à la poche (15) grâce à un ressort de traction (10),
**caractérisé en ce que**
la moitié supérieure (1) de logement est reliée de manière pivotante à la moitié inférieure (2) de logement,
le mécanisme agit sur un piston de compression (3) disposé dans le logement, entre lequel piston de compression et la moitié inférieure de logement est disposée la poche (15), le ressort de pression (10) précontraignant le levier oscillant dans sa position basculée correspondant à la position la plus basse du piston de compression, et
le levier oscillant (6) étant placé de manière rotative et déplaçable à une extrémité sur la moitié supérieure (1) de logement et accueillant de manière rotative à l'autre extrémité le piston de compression (3).

2. Dispositif selon la revendication 1, **caractérisé en ce que** le ressort de traction (10) s'accroche à une extrémité du levier oscillant (3) par l'intermédiaire d'un câble flexible (5).

3. Dispositif selon la revendication 2, **caractérisé en ce que** le câble flexible (5) est guidé au-dessus du côté supérieur du levier oscillant (6) et le côté supérieur est conformé avec un contour prédéterminé, en particulier en forme de toit, le câble étant dévié plus ou moins fortement en fonction de la position angulaire du levier oscillant (6).

4. Dispositif selon une des revendications précédentes, **caractérisé en ce qu'**il présente au moins deux leviers oscillants (6) agissant en sens contraire l'un par rapport à l'autre et comprenant des ressorts de traction (10) y afférents agissant en sens contraire, les leviers oscillants (6) ayant sur le piston de compression (3) un axe de rotation commun (13) sur leur autre extrémité respective.

5. Dispositif selon la revendication 4, **caractérisé en ce qu'**il présente deux leviers oscillants (6) situés à l'intérieur par rapport à l'axe de rotation commun (13), agissant dans une direction à partir de l'axe de rotation commun (13) et deux leviers oscillants (6) agissant en sens contraire avec des ressorts de traction (10) correspondants.

6. Dispositif selon une des revendications précédentes, **caractérisé en ce que** les leviers oscillants (6) peuvent être déplacés sur des nervures au moyen de rouleaux (4), les rouleaux (4) étant placés de manière rotative dans des évidements concaves complémentaires de la moitié supérieure (1) de logement dans leur position terminale.

7. Dispositif selon une des revendications précédentes, **caractérisé en ce qu'**il présente un logement plat.

8. Dispositif selon une des revendications précédentes, **caractérisé en ce que** le logement et le piston de compression (3) ont une forme approximativement rectangulaire.

9. Dispositif selon une des revendications précédentes, **caractérisé en ce que** le piston de compression (3) est conformé de manière concave en direction du bas dans le sens de son axe longitudinal pour s'adapter à la poche (15) contenant le liquide à administrer.

10. Dispositif selon une des revendications précédentes, **caractérisé en ce que** le logement présente au moins un passant de ceinture sur son côté arrière.

11. Dispositif selon une des revendications précédentes, **caractérisé en ce que** la fermeture présente au moins une languette de fermeture (8) disposée sur une moitié (1, 2) de logement, s'appliquant par l'extérieur sur l'autre moitié de logement lors de la fermeture et s'encliquetant dans l'autre moitié de logement.
